# EUROPEAN PATENT APPLICATION

(11) **EP 2 534 942 A1**
(43) Date of publication of application: **19.12.2012**
(21) Application number: 11170241.1
(22) Date of filing: 16.06.2011
(51) Int. Cl.: A01H 5/08, A01H 1/04, C12Q 1/68

(54) **Tomato plant absent in green staining**

(71) Applicant: Rijk Zwaan Zaadteelt en Zaadhandel B.V., 2678 KX De Lier (NL)
(72) Inventor: Vogelaar, Arie, 3328 ZC Dordrecht (NL); Dräger, Dörthe, 2635 JK Den Haag (NL); Verhoef, Rudolf, 2671 GK Naaldwijk (NL); Van Herwijnen, Zeger, 3043 HB Rotterdam (NL)
(74) Representative: Van Someren, Petronella F. H. M.

(57) **Abstract**

The present invention relates to a tomato plant (*Solanum lycopersicum* L.) comprising a genetic determinant that leads to the absence or reduction of green staining from the green vegetative parts of the plant upon contact, as compared to a plant not carrying the said genetic trait, which genetic determinant is obtainable from a tomato plant comprising said genetic determinant, representative seed of which was deposited with the NCIMB under deposit number NCIMB 41845. The said genetic determinant is preferably located on Linkage Group 9 between positions 1750800 bp and 4517648 bp, preferably between positions 1939546 bp and 2970346 bp, most preferably at about position 2454946 bp on a physical map.

## Description

The present invention relates to a tomato plant (*Solanum lycopersicum* L.) comprising a genetic determinant that leads to the absence or reduction of green staining from the green vegetative parts of the plant upon contact. The invention further relates to a marker and the use of the marker for identifying the presence of the genetic determinant that leads to the absence or reduction of green staining. The invention also relates to the seeds and progeny of such plants and to propagation material for obtaining such plants. Furthermore the invention relates to the use of the plants, seeds and propagation material that comprise the genetic determinant as germplasm in a breeding programme.

Tomato is a vegetable crop grown worldwide in all conditions and climates, both in protected cultivation or in open field. Growing tomato plants requires a lot of labour and attention, during which the plants are handled repeatedly by various persons. Handling the plants includes activities such as grafting, planting, pruning, winding, and of course harvesting.

Persons working in a tomato crop rapidly notice that with every contact of a green part of the tomato plant, a green substance comes off on the skin or any surface the contact is made with. The substance can also be yellowish in colour. Especially hands and clothes become covered with this substance, which after very intensive handling can result in almost black greenish stains on for example the hands. The main difficulty of this substance is that it is very hard to remove from the skin or clothes by normal washing. The green or yellowish substance results in green staining.

Another aspect of a tomato plant is the typical tomato smell that it emits. This can become very unpleasant when one is frequently exposed to a tomato crop.

The surface of the various plant parts of tomato (*Solanum lycopersicum*) is covered with trichomes, both non-glandular and glandular. Non-glandular trichomes are usually regarded as 'hairs' and do not produce, store, or secrete specific biochemical compounds.

Many biochemical compounds in tomato however are produced in glandular trichomes. A glandular trichome typically consists of a stalk, made up of one or more cells, and one or more glandular cells at the tip of the stalk that form the glandular head. Four different types of glandular trichomes are identified in tomato and related *Solanum* species, namely types I, IV, VI, and VII. These types differ in size and length of the stalks, and in number of secretory cells that form the glandular head (McDowell et al., Plant Physiology Vol. 155, 524-539 (2011)).

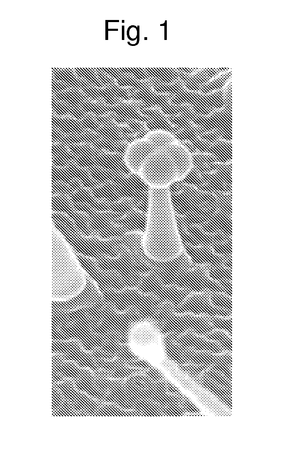

Type VI trichomes are composed of four disc cells, or glandular cells, at the end of a one- or two-celled stalk. The four disc cells form the glandular head (**Fig. 1**).

Biochemical compounds that are produced by the glandular trichomes in tomato comprise terpenoids, flavonoids, fatty acids, alkaloids, and acyl sugars such as acyl glucoses and acyl sucroses. These compounds are known to play important roles in attracting and repelling various insects, in determining susceptibility to certain diseases, and in aroma of the plant. However, many aspects of the roles of these metabolites are still unclear, and extensive research is ongoing to determine more precisely the functionality of glandular trichomes and the substances they excrete.

It is an object of the present invention to provide a tomato plant (*Solanum lycopersicum* L.) that carries a genetic determinant which leads to the absence or reduction of green staining from the green vegetative parts of the plant upon contact, as compared to a plant not carrying the said genetic trait. In this context the *"*genetic trait" is the phenotype in which green staining is absent or reduced. "Genetic determinant" is the underlying genetic element that causes the phenotypic trait. "Genetic trait" and "phenotypic trait" can be used interchangeably.

It is a further object of the present invention to provide a marker that can identify the genetic determinant leading to the absence or reduction of green staining from the green vegetative parts of the plant upon contact.

During the research that led to the present invention new tomato plants were created, that upon touching do not leave a green residue or green staining. The use of tomato plants of the invention leads to major advantages during tomato growing. The necessity to treat the skin with a substance in advance of handling tomato plants to prevent staining is removed. The use of protective clothing or gloves when working in a tomato crop is largely reduced. In addition, no rigorous methods to remove the staining after handling tomato plants are required anymore. The use of tomato plants of the invention will make tomato growing a lot more pleasant. Furthermore, the tomato plants of the invention do not give off a typical tomato-plant aroma.

The present invention thus provides a tomato plant (*Solanum lycopersicum*) comprising a genetic determinant which leads to the absence or reduction of green staining from the green vegetative parts of the plant upon contact, as compared to a plant not carrying the said genetic determinant.

In one embodiment, the genetic determinant that leads to the absence or reduction of green staining from the green vegetative parts of the plant upon contact is obtainable from a tomato plant comprising said genetic determinant, representative seed of which was deposited with the NCIMB under deposit number NCIMB 41845.

In one embodiment, the said genetic determinant is located on Linkage Group 9 between positions 1750800 bp and 4517648 bp, preferably between positions 1939546 bp and 2970346 bp, most preferably at about position 2454946 bp on a physical map.

In one embodiment, the tomato plant of the invention carrying the genetic determinant is obtainable by crossing a first tomato plant with a second tomato plant, wherein one of the said plants is grown from seeds as deposited with the NCIMB under deposit accession number NCIMB 41845, or a progeny plant thereof, and selecting, preferably in the F2 generation, for plants that show no or reduced green staining from the green vegetative parts of the plant upon contact, as compared to a plant not carrying the said genetic trait.

In one embodiment, one of the said plants used for crossing to obtain a tomato plant of the invention is carrying the said genetic trait that results in the absence or reduction of green staining from the green vegetative parts of the plant upon contact.

The genetic determinant that results in the trait of the invention preferably also leads to the absence of at least one of the four glandular cells, preferably the absence of two, three or all four glandular cell, that form the glandular heads of type VI trichomes.

In a preferred embodiment, the genetic determinant leads to the absence of green staining from the green vegetative parts of the plant upon contact.

In one embodiment, the genetic determinant that leads to the absence or reduction of green staining from the green vegetative parts of the plant upon contact is present in homozygous form. The trait of the invention is monogenic, i.e. caused by a single gene, and inherited in a recessive way. With respect to the trait of the invention, plants that carry the genetic trait can suitably be identified among descendants from a cross between a plant not carrying the trait, and a plant that does carry the said trait, by growing F2 plants from seeds that are the result from the initial cross and a selfing step, and selecting plants showing the desired trait. Selecting the plants can be done phenotypically by determining the reduction or absence of green staining, or can be done through identification of the genetic determinant, for example by means of the marker defined herein.

Determining absence or reduction of green staining is done in comparison to a control. Suitably, the control to determine the absence of green staining is any existing tomato plant. The control for determining reduction of green staining is preferably an isogenic tomato plant, i.e. a plant that is genetically identical to a plant of the invention, but does not carry the genetic determinant that results in the trait of the invention.

In one embodiment selecting the plants in the F2 can be done phenotypically by determining the absence of at least one, preferably two, three or all of the four glandular cells that form the glandular heads of type VI trichomes.

The glandular trichomes in cultivated tomato (*Solanum lycopersicum*) that are most abundant are type I and type VI. In research that led to the invention, a mutant tomato plant that did not show green staining from the vegetative plant parts was developed. The mutant tomato plant was also lacking the aroma that is typical for tomato plants. Plant growth and plant type of the new tomato plant were comparable to normal tomato plants. The mutant plant was not considered to be weak in growth or in plant habit.

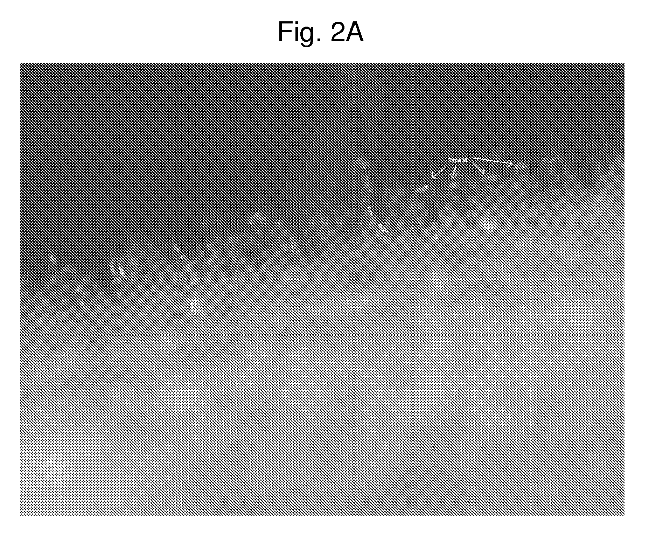

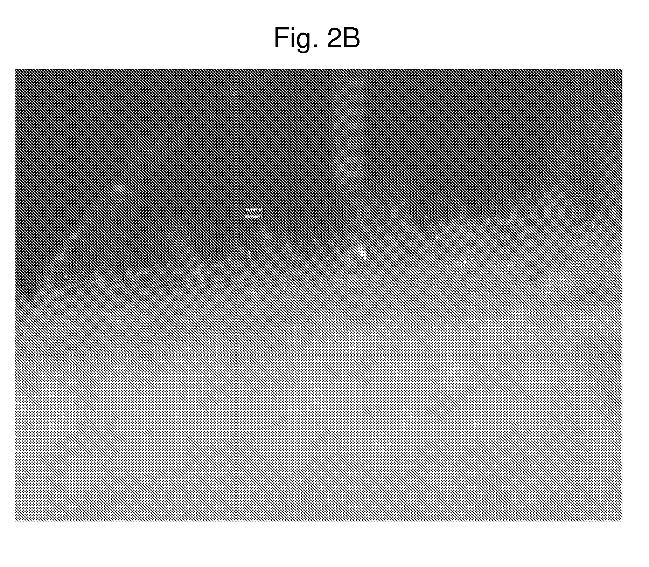

The new tomato plant was analysed for phenotypical characteristics. Surprisingly, the observation of the surface of green plant parts showed that wild type trichomes of type VI could not be detected in plants of the invention. The typical glandular heads of type VI trichomes, which are made up of four glandular cells, were not present among the trichomes (**Fig. 2B**). It was therefore concluded that the absence of glandular cells of type VI trichomes results in absence of green staining. The absence of functional type VI glands will understandably result in the same phenotypic trait. Malfunction of type VI trichomes, i.e. glands that do not function optimal, might result in absence or reduction of green staining as well.

The invention thus relates to plants that have modified type VI trichomes which modification leads to absent or non-functional glandular cells or glandular cells that do not function properly. The trait of having modified trichomes that lack all of the four glandular cells that make up the glandular head can for example be obtained from plants, representative seeds of which were deposited with the NCIMB under number NCIMB 41845 but the trait of lacking one, two, three or four glandular cells or having non-functional or mal-functioning glandular cells could also be obtained from other, yet unknown sources. According to the invention it was found that the absence or reduction of green staining is the result of such modified trichomes.

Further analysis of the new tomato plants was done to determine the presence of biochemical compounds. Measurements were done on three groups of volatiles: terpenes, sesquiterpenes, and aldehydes. The analysis showed that the trait of the invention resulted in a significant reduction of all volatiles that were analysed within these groups. Some volatiles could not be detected and are considered to be absent. The biochemical analysis is described in detail in **Example 5**.

Selection can be done on phenotype, i.e. determining the presence of the phenotypic trait, which is the absent or reduced green staining, or on the presence of the relevant genetic determinant. Selection can also be done by using one or more molecular markers. The use of molecular markers results in a reliable outcome, and can be done in a very early plant stage.

In one embodiment, the presence of the genetic determinant is preferably associated with the molecular marker characterized by SEQ.ID No:1 (**Table 1**).

It was found according to the invention that the genetic determinant leading to the trait of the invention is located on chromosome 9 of the tomato genome between the physical positions 1750800 bp and 4517648 bp.

In one embodiment the genetic trait can be identified by the presence of a molecular SNP marker. This SNP marker is located at 1750800 bp on a physical map and is characterized by SEQ ID No:1 (**Table 1**).

In one embodiment the genetic determinant is located between the physical positions 1939546 bp and 2970346 bp on a physical map.

In a preferred embodiment the genetic determinant is located at the physical position 2454946 bp on a physical map. The physical map used for positioning the genetic determinant is the public *Solanum lycopersicum* map version SL2.30.

The invention furthermore relates to a cell of a tomato plant as claimed. Such cell may be either in isolated form or may be part of the complete tomato plant or parts thereof and then still constitutes a cell of the invention because such a cell harbours in its genetic constitution the genetic information that leads to the characteristics that define the tomato plant of the invention. Each cell of tomato plants of the invention carries the genetic information that leads to phenotypic expression of said trait.

The invention also relates to tissue of a plant as claimed. The tissue can be undifferentiated tissue or already differentiated tissue. Undifferentiated tissues are for example stem tips, anthers, petals, pollen and can be used in micropropagation to obtain new plantlets that are grown into new plants of the invention.

The invention according to a further aspect thereof relates to seeds of a plant as claimed. Although the seeds do not show the genetic trait of the tomato plant of the invention, they harbour the genetic information that when a plant is grown from the seeds makes this plant a plant of the invention.

The invention also relates to progeny of the plants, cells, tissues and seeds of the invention. Such progeny can in itself be plants, cells, tissues or seeds.

As used herein the word "progeny" is intended to mean the first and all further descendants from a cross with a plant of the invention that comprises a genetic determinant that leads to absence or reduction of green staining from the green vegetative parts of the plant upon contact. Progeny of the invention are descendants of any cross with a plant of the invention that carries the trait that leads to absence or reduction of green staining.

"Progeny*"* also encompasses plants that carry the trait of the invention and are obtained from other plants or progeny of plants of the invention by vegetative propagation or multiplication.

The invention thus further relates to seed of the claimed plant and to parts of the plant that are suitable for sexual reproduction. Such parts are for example selected from the group consisting of microspores, pollen, ovaries, ovules, embryo sacs and egg cells. In addition, the invention relates to parts of the plant that are suitable for vegetative reproduction, in particular cuttings, roots, stems, cells, and protoplasts.

According to a further aspect thereof the invention provides a tissue culture of the claimed plant. The tissue culture comprises regenerable cells. Such tissue culture can be derived from leaves, pollen, embryos, cotyledons, hypocotyls, meristematic cells, roots, root tips, anthers, flowers, seeds and stems.

The invention furthermore relates to hybrid seed and to a method of producing hybrid seed comprising crossing a first parent plant with a second parent plant and harvesting the resultant hybrid seed, wherein said first parent plant and/or said second parent plant is the plant as claimed.

The invention also relates to inbreds and doubled haploids of tomato plants of the invention.

In one embodiment, the invention relates to tomato plants of the invention that carry the genetic determinant which leads to the absence or reduction of green staining from the green vegetative parts of the plant upon contact, and having acquired said determinant by introduction of the genetic information that is responsible for the trait from a suitable source, either by conventional breeding, or genetic modification, in particular by cisgenesis or transgenesis. Cisgenesis is genetic modification of plants with a natural gene, coding for an (agricultural) trait, from the crop plant itself or from a sexually compatible donor plant. Transgenesis is genetic modification of a plant with a gene from a non-crossable species or a synthetic gene.

The invention also relates to the germplasm of plants of the invention. The germplasm is constituted by all inherited characteristics of an organism and according to the invention encompasses at least the trait of the invention. The germplasm can be used in a breeding programme for the development of tomato plants that are lacking in or have a reduction in green staining from the green vegetative parts of the plant upon contact.

The invention also relates to a tomato fruit that is produced by a plant of the invention. The invention further relates to a food product, comprising the fruit of a tomato plant as claimed, or parts thereof. The invention also relates to a food product in processed form.

The term 'genetic determinant' as used herein encompasses a gene or allele. These terms are used interchangeably.

A genetic determinant can be identified by the use of a molecular marker. A genetic determinant can alternatively be identified by the position on a genetic map, or by indication of the location on a linkage group or chromosome. When a genetic determinant is not linked to a specific molecular marker any longer, but its position on a chromosome as defined on a genetic map is unaltered, this genetic determinant is still the same as when it was linked to the molecular marker. The genetic trait that it confers is therefore also still the same.

The 'genetic trait' is the trait or characteristic that is conferred by the genetic determinant. The genetic trait can be identified phenotypically, for example by performing a bio-assay. However, also plant stages for which no phenotypic assay can be performed do carry the genetic information that leads to the genetic trait. 'Trait' or 'phenotypic trait' can be used instead of 'genetic trait'.

In the absence of molecular markers, equivalence of genetic determinants can be determined by an allelism test. To perform an allelism test, material that is homozygous for the known determinant is crossed with material that is homozygous for the genetic determinant to be tested. When no segregation for the trait to be observed is present in the F2 of the cross, the genetic determinants have been proven to be the same.

When more than one gene is responsible for a certain trait, and an allelism test is done to determine equivalence, the skilled person doing the test has to make sure that all relevant genes are present homozygously for the test to work properly.

Genetic maps can vary according to the method by which they are assembled. A person skilled in the art knows how to compare and combine genetic maps, whereby differences between genetic maps can be eliminated or minimized. Information from one genetic map can therefore be transferred or translated to another genetic map. The positions as used herein are physical positions based on the public physical map of the tomato genome SL2.30 (version of August 2010, http://solgenomics.net/organism/ solanum_lycopersicum/genome).

### DEPOSIT

Seeds of *Solanum lycopersicum* 11R-5000 that comprise the genetic determinant of the invention which leads to the absence or reduction of green staining were deposited with NCIMB Ltd, Ferguson Building, Craibstone Estate, Bucksburn, Aberdeen AB21 9YA, UK on 13 June 2011 under deposit accession number NCIMB 41845.

### FIGURES

**Figure 1**: Type VI trichome *Solanum lycopersicum* (Kang et al. J. Exp. Bot 61(4), 1053-1064, detail Suppl. Material, (2010))
**Figure 2**: A: Wildtype TR306-1 type VI trichomes with glandular head. B: mutant 3432-1 type VI trichomes with glandular heads absent.
**Figure 3**: Measurements of various terpenes in plants of the invention (Toll/013) as compared to normal wild-type tomato plants ('all others'); n.d. = not detected
**Figure 4**: Measurements of various sesquiterpenes in plants of the invention (Toll/013) as compared to normal wild-type tomato plants ('all others'); n.d. = not detected
**Figure 5**: Measurements of various aldehydes in plants of the invention (Toll/013) as compared to normal wild-type tomato plants ('all others'); n.d. = not detected

The invention will be further illustrated in the Examples that follow.

### EXAMPLES

### EXAMPLE 1

### Creation of tomato plants of the invention

Seeds of two tomato breeding lines, TR306 and T029, were treated with ems (ethyl methane sulfonate) by submergence of approximately 10.000 seeds into an aerated solution of 0.5% (w/v) ems during 24 hours at room temperature.

The treated seeds were germinated and the resulting plants were grown in a greenhouse to produce M2 seeds.

After maturation, M2 seeds were harvested and bulked in one pool. The resulting pool of M2 seeds was used as starting material to identify individual M2 plants that were absent or reduced in green staining upon contact with the green plant parts.

The efficacy of the genetic modification procedure was assessed by determining the occurrence of bleached plants, which is indicative for chlorophyll loss due to modifications in genes directly or indirectly involved in the formation or accumulation of chlorophyll.

### EXAMPLE 2

### Phenotypic identification of a plant of the invention

M2 tomato seeds were germinated in soil and grown to small plantlets. Subsequently, approximately 7000 randomly chosen plants were transferred for a seedling screen.

Plants were touched to establish whether upon making contact the green vegetative parts would leave a yellowish or greenish substance on the skin. Three mutant plants were identified that, upon making contact, did not leave green staining. In addition, it was observed that the mutant plants did not give off the aroma that is typical for tomato plants. The mutant plants that were developed with this trait have the identification codes M2-2517, M2-4416, and M2-T029-mut. Seeds from M2-2517 were multiplied and resulted in population 11R-5000, which were deposited with the NCIMB under number NCIMB 41845.

The green plant parts of the mutant plants were visually observed by use of a binocular, and compared with normal wild type (WT) tomato plants. The microscopic observation showed that the glandular heads of type VI trichomes, which are made up of four glandular cells, were not present among the glands of the mutant tomato plants. It was therefore concluded that the absence of type VI glands resulted in absence of green staining.

### EXAMPLE 3

### Transfer of the trait of the invention to other tomato plants

Plants of the invention were crossed with wild type (WT) tomato plants, which do not carry the trait of the invention. The resulting F1 from this cross had the same phenotype as the WT plant, i.e. it did show green staining upon establishing contact. When the trichomes were observed, the F1 was found to have normal type VI trichomes, with the four glandular cells to form the glandular head.

The F2 segregated in a manner that corresponds with a monogenic recessive inheritance of the trait of the invention. Both the absence of green staining and the absence of four glandular cells of type VI trichomes segregated in the same way.

The trait of the invention could be brought into a wild type tomato plant by crossing the wild type plant with a plant of the invention and selecting for the desired phenotype, either by selection on absence of green staining, or by selection on absence of four glandular cells of type VI trichomes.

### EXAMPLE 4

### QTL mapping and marker development

A population of 90 F2 plants, as obtained from **Example 3,** were genotyped with 196 SNP markers. 179 of these SNP markers were reliably polymorphic and were used to do a QTL analysis for the absence of green staining. As expected for a monogenic trait, one genetic determinant was located, positioned on chromosome 9. This genetic determinant explained 88% of the variance of the trait.

The SNP marker that correlated most closely to the gene is presented in **Table 1**. This molecular marker can be used to identify the presence of the genetic determinant in plants grown from seeds as deposited under NCIMB number 41845.

### EXAMPLE 5

### Biochemical analysis of plants of the invention

Plants of the invention were analysed for a large number of volatiles, and compared with a tomato plant that did not contain the genetic determinant of the invention. A tomato plant to be measured was placed in a plastic bag for 10 minutes. Next, the plants were touched to allow the release of the relevant compounds to be analysed. Subsequently Solid Phase Microextraction (SPME) was performed for 15 minutes at room temperature.

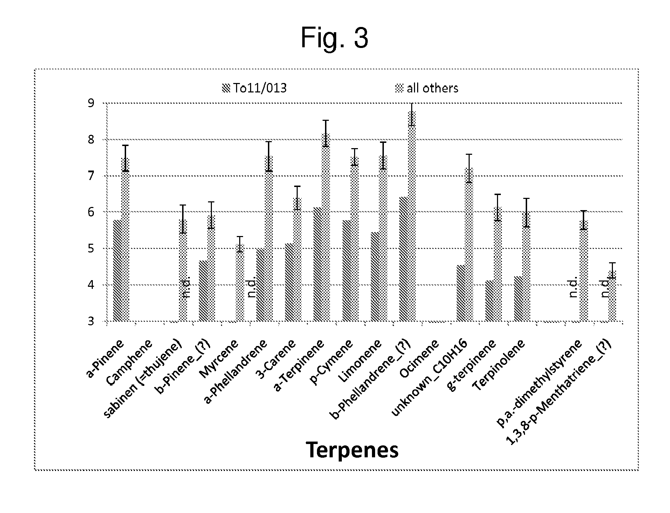

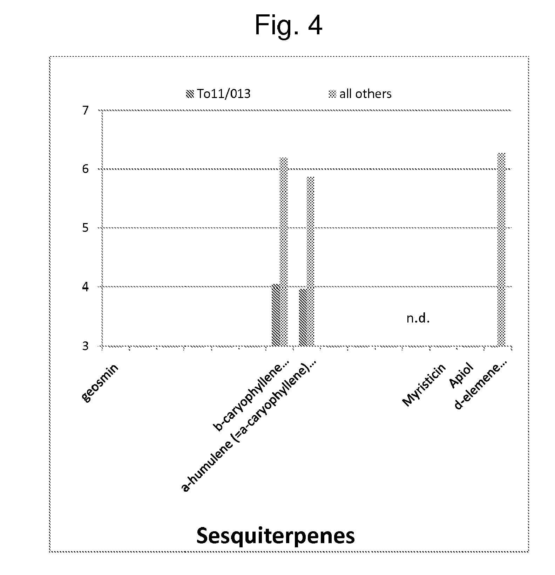

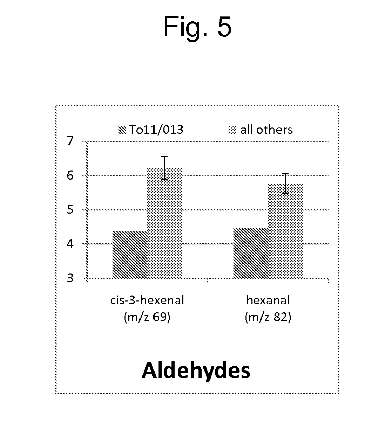

Three groups of compounds were analysed by using GC-MS: terpenes, sesquiterpenes, and aldehydes. Remarkably, for all the volatiles within these groups it was found that they were significantly reduced as compared to the wild type plant (**Fig. 3-5**). Some volatiles were even below detection level and therefore considered to be absent.

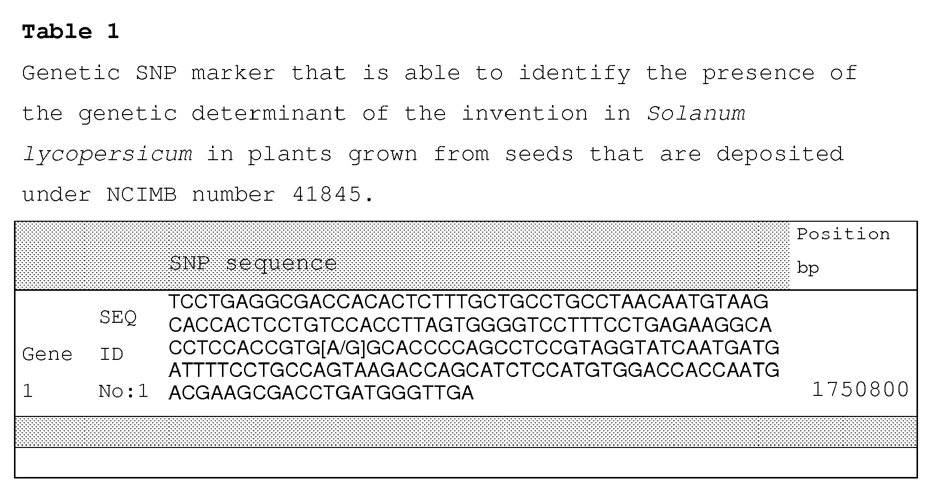

## Claims

1. Tomato plant (*Solanum lycopersicum* L.) comprising a genetic determinant that leads to the absence or reduction of green staining from the green vegetative parts of the plant upon contact, as compared to a plant not carrying the said genetic trait, which genetic determinant is obtainable from a tomato plant comprising said genetic determinant, representative seed of which was deposited with the NCIMB under deposit number NCIMB 41845.

2. Tomato plant as claimed in claim 1, wherein the said genetic determinant is located on Linkage Group 9 between positions 1750800 bp and 4517648 bp, preferably between positions 1939546 bp and 2970346 bp, most preferably at about position 2454946 bp on a physical map.

3. Tomato plant as claimed claim 1 or 2, obtainable by crossing a first tomato plant with a second tomato plant, wherein one of the said plants is grown from seeds as deposited with the NCIMB under deposit accession number NCIMB 41845, or a progeny plant thereof, and selecting, preferably in the F2 generation, for plants that are absent or reduced in green staining from the green vegetative parts of the plant upon contact, as compared to a plant not carrying the said genetic trait.

4. Tomato plant as claimed in claim 1 or 2, obtainable by crossing a first tomato plant with a second tomato plant, wherein one of the said plants is carrying the said genetic trait that results in the absence or reduction of green staining from the green vegetative parts of the plant upon contact, and selecting, preferably in the F2 generation, for plants that are absent or reduced in green staining from the green vegetative parts of the plant upon contact, as compared to a plant not carrying the said genetic trait.

5. Tomato plant as claimed in any of the claims 1 to 4, wherein the genetic determinant leads to the absence of the four glandular cells that form the glandular heads of type VI trichomes.

6. Seed of a tomato plant as claimed in any one of the claims 1-5, wherein the plant that can be grown from the seed comprises the genetic determinant as defined in claim 2.

7. Progeny of a tomato plant as claimed in any one of the claims 1-5 or of tomato seed as claimed in claim 6, comprising the genetic determinant as defined in claim 2.

8. Propagation material suitable for producing a plant as claimed in any one of the claims 1-5 or seed as claimed in claim 6.

9. Propagation material as claimed in claim 8, which propagation material is formed by parts of the plant that are suitable for
- sexual reproduction, in particular microspores, pollen, ovaries, ovules, embryo sacs and egg cells.
- vegetative reproduction, in particular cuttings, roots, stems, cells and protoplasts, tissue cultures of regenerable cells.
- tissue culture comprise leaves, pollen, embryos, cotyledons, hypocotyls, meristematic cells, roots, root tips, microspores, anthers, flowers, seeds and stems.

10. Propagation material as claimed in claim 8 or 9, wherein the plant produced from the propagation material comprises the genetic determinant as defined in claim 2.

11. Tomato plant as claimed in any of the claims 1-5 and 7, seed as claimed in claim 6, or propagation material as claimed in any of the claims 8-10, wherein the said genetic determinant can be identified by a molecular marker **characterized by** SEQ ID No. 1.

12. Tomato plant as claimed in claim 11, wherein the genetic determinant is located in the genome of seeds of which a representative sample was deposited with the NCIMB under deposit number NCIMB 41845 on Linkage Group 9 between positions 1750800 bp and 4517648 bp, preferably between positions 1939546 bp and 2970346 bp, most preferably at about position 2454946 bp on physical map SL2.30.

13. A tomato fruit of a plant as claimed in any one of the claims 1 to 5.

14. Food product, comprising the tomato fruit as claimed in claim 15, or parts thereof, optionally in processed form.

15. Use of a plant as claimed in any one of the claims 1-5, 7 and 13, or plants produced from the seed of claim 6, or from the propagation materials as claimed in any one of the claims 8-10 as germplasm in a breeding programme for the development of tomato plants that are absent or reduced in green staining from the green vegetative parts of the plant upon contact, as compared to a plant not carrying the said genetic trait and/or lack at least one of the four glandular cells that form the glandular heads of type VI trichomes.
